(19) Europäisches Patentamt
European Patent Office
Office européen des brevets

(11) Publication number: **0 001 854**
**A2**

(12) **EUROPEAN PATENT APPLICATION**

(21) Application number: 78200261.2

(22) Date of filing: 26.10.78

(51) Int. Cl.[2]: C 07 C 67/48
C 07 C 69/52, C 07 C 69/24

(30) Priority: 28.10.77 US 846300

(43) Date of publication of application:
16.05.79 Bulletin 79/10

(84) Designated contracting states:
BE CH DE FR GB LU NL

(71) Applicant: THE PROCTER & GAMBLE COMPANY
301 East Sixth Street
Cincinnati Ohio 45217(US)

(72) Inventor: Logan, Ted Joe
8880 Livingstone Road
Colerain Twp. Hamilton County(US)

(74) Representative: Ernst, Hubert et al,
PROCTER & GAMBLE EUROPEAN TECHNICAL CENTER
Temselaan 100
B-1820 Strombeek-Bever(BE)

(54) **Process for separating unsaturated and saturated fatty acid esters.**

(57) A process for separating mixtures of fatty acid esters by degree of unsaturation by contacting said mixtures with an X or Y zeolite, thereby selectively absorbing the unsaturated esters onto the zeolite.

EP 0 001 854 A2

# PROCESS FOR SEPARATING UNSATURATED AND SATURATED FATTY ACID ESTERS

The field to which this invention pertains is the separation of fatty acid esters by degree of unsaturation. More specifically the invention relates to a process of separating esters of unsaturated fatty acids from esters of saturated fatty esters, by contacting said mixture with a solid absorbent which has a higher affinity for the unsaturated fatty acid esters.

It is often important to separate fatty materials such as fatty acid esters into fractions of different degrees of unsaturation since there are specific uses for fatty materials having, respectively, higher or lower degrees of unsaturation. The saturated fatty acid esters, for example, are especially useful in the manufacture of lubricants, cosmetics,

resins, plasticizers and wetting agents. For such specific purposes, a fatty acid ester product becomes more valuable as the proportion of saturated components is increased in the product.

The use of solid adsorbents such as zeolites (also called molecular sieves) to separate olefinic hydrocarbons from paraffinic hydrocarbons is well known. For example, U.S. Patents 3,265,750, issued August 9, 1966, to Peck et al., and 3,510,423, issued May 5, 1970, to Neuzil et al., describe the use of X and Y zeolites to conduct such separations.

Heretofore, the most common means of producing fatty acid esters of specified levels of saturation or unsaturation has been by fractional crystallization of the fatty acids to produce more saturated and less saturated fractions of the acids, and then reacting these fractions with alcohols to produce the esters. The present process provides a means of separating the esters themselves, according to degree of unsaturation, and provides better separation than has been possible by fractional crystallization of the acids. At the same time, the present process avoids the large consumption of energy inherent in using fractional crystallization processes.

## SUMMARY OF THE INVENTION

It is a broad objective of the present invention to provide a process for separating mixtures of saturated and unsaturated fatty acid esters into fractions which are enriched, respectively, in the unsaturated components and the saturated components, in comparison to the original

mixture. In a more specific sense, it is an objective of the invention to provide a process for separating methyl esters of fatty acids by degree of unsaturation, and more particularly, separating methyl oleate, methyl linoleate or methyl linolenate, or all three, from methyl palmitate or methyl stearate, or both.

The zeolites used as the adsorbents herein have a preferential affinity for the unsaturated components of the fatty acid ester mixture. Accordingly, in the process of the invention, the fraction of the fatty acid ester feedstock which is adsorbed on the zeolite becomes enriched in the unsaturated components, while the fraction which remains unadsorbed becomes enriched in the saturated components.

## DESCRIPTION OF THE INVENTION

The present invention comprises a process for separating a lower alkyl ester of an unsaturated fatty acid from a mixture comprising a lower alkyl ester of an unsaturated fatty acid and a lower alkyl ester of a saturated fatty acid, which process comprises contacting, at a temperature of about 25°C or higher and a pressure within the range of from about one atmosphere to 500 psig., said mixture with a zeolite adsorbent comprising an X or Y zeolite containing at exchangeable cationic sites at least one cation selected from the group consisting of cations of metals of Group IA of the Periodic Table of Elements, magnesium, calcium, strontium, barium, silver, gold, zinc, cadmium, nickel and mercury, and combinations thereof, thereby selectively adsorbing unsaturated fatty acid esters on said sieve. The process is preferably conducted at temperatures above 25°C, and more preferably

between about 25°C and about 150°C. Preferably, the mixture of fatty acid esters is in the form of a liquid phase when being contacted with the zeolite adsorbent.

The fatty acid esters which comprise the feedstock which is to be separated according to the present process are reaction products of a fatty acid and a lower ($C_1$-$C_4$) alcohol. Generally, the alcohol is a monohydric short chain alcohol such as methanol, ethanol, propanol or butanol. From a commercial standpoint the methyl esters are especially important and for purposes of simplicity the present process will be described primarily with reference to methyl esters, although it should be kept in mind that other esters of fatty acids can also be separated by the process.

The fatty acids from which the esters are made comprise a large group of monocarboxylic aliphatic acids which can be saturated (i.e., no double bonds) or unsaturated (i.e., containing one or more double bonds). The most common of these acids contain from about 6 to about 22 carbon atoms in the aliphatic chain and 0, 1, 2 or 3 sites of double bond unsaturation.

Examples of individual esters are methyl stearate, methyl caprate, methyl laurate, methyl palmitate, methyl lauroleate, methyl myristoleate, methyl oleate, methyl eicosanate, methyl linoleate, methyl linolenate, methyl oleostearate and methyl elaidate. Methyl esters of fatty acids are normally produced commercially by methanolysis of naturally occurring glycerides, rather than by reaction with fatty acids per se. The fatty chains within these glycerides vary within the same glyceride molecule, as well as between glyceride molecules, in chain length and in degree of

unsaturation; thus the methyl esters so produced are also mixtures. For example, typical mixtures of methyl esters obtained from the methanolysis, respectively, of soybean oil and beef tallow, have the following compositions:

TABLE I

Composition of Fatty Methyl Esters (% by Weight)

| | From Soybean Oil | From Tallow |
|---|---|---|
| Saturates | | |
| Myristate or lower | Tr.-0.5 | 2-8 |
| Palmitate | 7-11 | 24-37 |
| Stearate | 2-6 | 14-29 |
| Eicosanate or higher | 0.3-3 | Tr.-1.2 |
| Unsaturates | | |
| Palmitoleate or lower | Tr.-1 | 2.3-3.3 |
| Oleate | 15-33 | 40-50 |
| Linoleate | 43-56 | 1-5 |
| Linolenate | 5-11 | - |

The fatty acid esters used as feedstock in the present process generally contain from 7 to about 26 carbon atoms per molecule.

Feed mixtures which are charged to the present process then will be those comprising an ester of an unsaturated fatty acid and an ester of a saturated fatty acid, with the object of the process being to separate the mixture into saturates and unsaturates. Preferred as feedstocks for the present process are methyl esters of such fatty acids. The feed mixtures will typically contain one or more unsaturated fatty acid esters, which may be monoethenoid, diethenoid, triethenoid, etc., or mixtures thereof, and one or more saturated fatty acid esters. An example of a feed mixture is one containing: 1 wt. % $C_{14}$ and lower saturated compounds; 28 wt. % methyl palmitate; 19 wt. % methyl stearate; 42 wt. % methyl oleate; 9 wt. % methyl linoleate; and 1% methyl linolenate and higher saturates and unsaturates. Except for the small amount of $C_{14}$ and lower material and the small amount of linolenate and higher saturate and unsaturate material, such feed mixture consists of $C_{16}$ and $C_{18}$ methyl esters of fatty acids and more specifically consists of two saturated fatty acid esters, one monoethenoid unsaturated fatty acid ester and one diethenoid unsaturated fatty acid ester. Feed mixtures which can be charged to the present process may contain, in addition to fatty acid esters, a diluent material, that is not adsorbed by the adsorbent and which is preferably separable from the fatty acid esters which are separated by the process, e.g., by distillation. Paraffinic hydrocarbons are examples of suitable diluents. Normal hexane, 2,2,4-trimethyl pentane and normal octane are specific examples of paraffinic hydrocarbons that can be used as diluents, and can easily be distilled from the fatty acid esters. When a diluent is employed the concentration of diluent in the mixture of diluent and fatty acid esters will generally be from about 10% to about 98% by weight, with the remainder being fatty acid esters.

Due to the high affinity of the zeolite adsorbent for the unsaturated esters, the feed material, upon contact with the zeolite, becomes separated into a raffinate, enriched in the saturated components, and an extract (on the adsorbent) enriched in unsaturates. The adsorbent and feed are kept in contact for a sufficient time to allow the desired degree of separation to occur. The degree of separation increases from the moment of initial contact up to the time when equilibrium is reached, which can typically take up to about one hour. Degree of separation can be determined by analysis of the raffinate, using standard gas-liquid chromatographic techniques. If further enrichment of the raffinate is desired beyond that which can be obtained in a single contact, the raffinate can be subjected to one or more additional contacts with additional zeolite. If a diluent (e.g., paraffinic hydrocarbon) has been used, the fatty acid esters in the raffinate can be separated from the diluent (e.g., by distillation).

The adsorbents which are used in the process of the present invention are the crystalline aluminosilicates designated as X and Y zeolites. They are also referred to in the art as X and Y molecular sieves. These materials are well known and have been described, respectively, in U.S. Patents 2,882,244, Milton, issued April 14, 1959, and 3,130,007, Breck, issued April 21, 1964, both incorporated herein by reference.

Zeolite X has the formula:

$$0.9 \pm 0.2\ M_{\frac{2}{n}}O:Al_2O_3:(2.5 \pm 0.5)SiO_2:xH_2O$$

wherein "M" represents a metal cation, "n" is its valence and "x" may be any value up to 8, depending on the identity of the metal and the degree of hydration of the crystal.

Zeolite Y has the formula:

$$0.9 \pm 0.2\ M_{\frac{2}{n}}O:Al_2O_3:wSiO_2:xH_2O$$

wherein "M" represents a metal cation, "n" is its valence, "w" is a value greater than 3 up to about 6 and "x" may be a value up to about 9, depending on the identity of M, and the degree of hydration of the crystal.

The position of "M" in the zeolite crystalline structure is normall referred to as an exchangeable cationic site.

Zeolites X and Y are normally produced and sold in the sodium form, i.e., "M" in the above formulas is sodium. If it is desired to substitute other metal ions for sodium at the exchangeable cationic sites, this can be accomplished by conventional cation exchange techniques, e.g., treating the sodium sieve with an aqueous solution of a salt of the desired cation.

The X and Y zeolites for use in the process of the present invention can have any of the following metals at the exchangeable cationic sites: the metals of Group IA of The Periodic Table of Elements (e.g., lithium or sodium) magnesium, calcium, strontium, barium, silver, gold, zinc, cadmium, mercury and nickel, or combinations thereof. The Group IA metals, especially sodium, are preferred.

The zeolites, as produced and sold commercially, normally contain, in addition to the actual zeolite, an inert diluent such as clay. Typical commercial material contains about 20% by weight clay and about 80% by weight zeolite.

The zeolites are available commercially from The Linde Company, Tonawanda, New York.

It is generally preferred that the zeolite adsorbent used in the process herein have a water content of 0% to about 10% by weight of the zeolite. A given moisture level can be achieved by heating the zeolite in an oven to constant weight to drive off all water, and then rehydrating the zeolite with the desired amount of water. The adsorbent used in the present process can have a particle size range of from about 20 to about 80 mesh (Standard U.S. Mesh) and will preferably have a particle size within the range of about 20 to 40 mesh.

In the present process the contact between the adsorbent and fatty acid ester feedstock may be effected in various ways. A mixture of feedstock and adsorbent may be slurried together, followed by a filtering-off of the raffinate fraction. The feedstock can be fed to a fixed bed of adsorbent, with the raffinate being collected at the exit from the bed. The process can also be conducted in continuous simulated moving bed manner such as that described for separating olefins and paraffins in U.S. Patent 3,510,423, issued May 5, 1970. When using a simulated moving bed operation, selection of an appropriate desorbent solvent system is important to obtain effective utilization of the process. For separating saturated and unsaturated fatty acid esters on X or Y zeolite adsorbents in such an operation, a 50%/50% by volume mixture of hexane/ diisopropyl ether is a suitable desorbent solvent system.

The invention will be illustrated by the following example.

EXAMPLE I

This example demonstrates the separation of a saturated fatty acid ester (methyl stearate) from an unsaturated fatty acid ester (methyl oleate), using a Y-Type molecular sieve.

The Y sieve, as purchased from Linde Company of Tonawanda, New York, was in the sodium form (i.e., the cation exchangeable sites were occupied by sodium) and consisted of about 20% by weight clay and 80% by weight zeolite.

The adsorbent was ground to a particle size range of 20-40 mesh and dried at 300°C for 2 hours. Three 20 gram portions were then weighed into flasks. 24 grams of silver nitrate were added to the first flask and 4.4 grams of silver nitrate were added to the second flask. 150 ml of distilled water was then added to all three flasks and they were shaken for one hour. The contents of the three flasks were then filtered and washed with water. The combined filter solution and washings from each flask were then analyzed for silver and the amount exchanged onto the sieve was determined by difference. The sieves were then dried overnight at 300°C and stored under vacuum.

Samples of the three prepared sieve materials were weighed into vials the weights being chosen so as to provide that each vial contained about 500 mg of actual zeolite sieve, calculated on the basis of sodium Y sieve. The compositions of the individual samples are shown in the table below.

4 ml of 2,2,4-trimethyl pentane (diluent) and a stir bar were added to each vial. 0.75 ml methyl oleate

(200.3 mg/ml concentration in 2,2,4-trimethyl pentane) and 0.75 ml methyl stearate (208 mg/ml concentration in 2,2,4-trimethyl pentane) were then added to each vial, the vials were purged with nitrogen and sealed, and the contents were stirred at 90°C for 60 minutes. 1 ml octacosane (gas chromatography internal standard) was then added to each vial and the contents were filtered on Whatman #40 filter paper and washed with 2,2,4-trimethyl pentane. The filtered solution (combined with wash solution) was then analyzed for methyl oleate and methyl stearate by gas chromatography. The amount of each ester adsorbed onto the sieve was then calculated by difference. The results are shown in the table below.

TABLE II

Separation of Methyl Esters on Y-Type Molecular Sieve

| Sample | mg of Sample | mg As Dry Na Sieve | mg Clay | mg Ag. | mg Me Oleate Added | mg Me Stearate Added | mg Me Stearate On Sieve | mg Me Oleate On Sieve | mg Me Stearate In Soln. | mg Me Oleate In Soln. |
|---|---|---|---|---|---|---|---|---|---|---|
| A | 837.4 | 491 | 123 | 223 | 150.2 | 156.0 | 12 | 100.2 | 144 | 50 |
| B | 704.5 | 496 | 124 | 85 | 150.2 | 156.0 | 12 | 99.2 | 144 | 51 |
| C | 606.7 | 486 | 121 | 0 | 150.2 | 156.0 | 19 | 70.2 | 137 | 80 |

The data show that in all instances a separation of methyl oleate from methyl stearate has occurred in that, compared to the original feed, there is a higher ratio of methyl oleate to methyl stearate on the sieve and a lower ratio of methyl oleate to methyl stearate in the liquid.

Effective separations can also be obtained when the sodium ion in sodium Y zeolite is exchanged for other Group IA ions, magnesium, calcium, strontium, barium, gold, zinc, cadmium, mercury or nickel, instead of silver, or when the sieve is an X sieve instead of a Y sieve.

WHAT IS CLAIMED IS :

1. A process for separating a lower alkyl ester of an unsaturated fatty acid from a mixture comprising a lower alkyl ester of an unsaturated fatty acid and a lower alkyl ester of a saturated fatty acid which process comprises contacting, at a temperature of at least about 25°C and a pressure within the range of from about atmospheric to about 500 psig., said mixture with an adsorbent comprising an X or Y zeolite containing at exchangeable cationic sites at least one cation selected from the group consisting of cations of Group IA of The Periodic Table of Elements, magnesium, calcium, strontium, barium, silver, gold, zinc, cadmium, mercury and nickel, and combinations thereof, thereby selectively adsorbing said ester of an unsaturated fatty acid.

2. The process of claim 1 further characterized in that said adsorbent contains sodium cations at the exchangeable cationic sites.

3. The process of claim 1 further characterized in that it is effected in the liquid phase.

4. The process of claim 1 further characterized in that said ester of an unsaturated fatty acid and said ester of a saturated fatty acid each contain from about 7 to about 26 carbon atoms.

5. The process of claim 4 further characterized in that said ester of an unsaturated fatty acid and said ester of a saturated fatty acid are methyl esters.

6. The process of claim 5 further characterized in that said ester of a saturated fatty acid ester is methyl palmitate or methyl stearate.

7. The process of claim 5 further characterized in that said ester of an unsaturated fatty acid is methyl oleate.

8. The process of claim 5 further characterized in that said ester of an unsaturated fatty acid is methyl linoleate.